# EUROPÄISCHE PATENTSCHRIFT

(11) **EP 0 657 430 B1**
(45) Veröffentlichungstag und Bekanntmachung des Hinweises auf die Patenterteilung: **02.06.1999**
(21) Anmeldenummer: 94118671.0
(22) Anmeldetag: 28.11.1994
(51) Int. Cl.: C07D 211/90, C07C 255/56, A61K 31/44

(54) **Isopropyl-(2-methoxyethyl)-4-(2-chlor-3-cyano-phenyl)-1,4-dihydro-2,6-dimethyl-pyridin-3,5-dicarboxylat**
Isopropyl-(2-methoxyethyl)-4-(2-chloro-3-cyano-phenyl)-1,4-dihydro-2,6-dimethyl-piridine-3,5-dicarboxylate
Isopropyl-(2-méthoxyéthyl)-4-(2-chloro-3-cyano-phényle)-1,4-dihydro-2,6-diméthyl-pyridine-3,5-carboxylate

(30) Priorität: 10.12.1993 DE 4342194; 13.07.1994 DE 4424677
(43) Veröffentlichungstag der Anmeldung: 14.06.1995
(73) Patentinhaber: BAYER AG, 51368 Leverkusen (DE)
(72) Erfinder: Meier, Heinrich Dr., Higashi-Nada-Ku, Kobe 658 (JP); Hartwig, Wolfgang Dr., Stamford, Connecticut 06903 (US); Junge, Bodo Dr., D-42399 Wuppertal (DE); Schohe-Loop, Rudolf Dr., D-42327 Wuppertal (DE); Gao, Zhan Dr., Beijing 100016 (CN); Schmidt, Bernard Dr., D-51789 Lindlar (DE); de Jong, Maarten Dr., D-51491 Overath (DE); Schuurman, Teunis Dr., D-53797 Lohmar (DE)

(56) Entgegenhaltungen:
- EP-A- 0 451 654
- EP-A- 0 494 816
- EP-A- 0 525 568

## Beschreibung

Die vorliegende Erfindung betrifft die neue Verbindung rac-Isopropyl-(2-methoxyethyl)-4-(2-chlor-3-cyano-phenyl)-1,4-dihydro-2,6-dimethylpyridin-3,5-dicarboxylat und dessen reine Enantiomere, Verfahren zu ihrer Herstellung und ihre Verwendung als Arzneimittel, insbesondere zur Behandlung von cerebralen und neuronalen Erkrankungen sowie ein neues Zwischenprodukt zu ihrer Herstellung.

Es ist bereits bekannt, daß einige 1,4-Dihydropyridine wie z.B. Nimodipin eine cerebrale Wirksamkeit besitzen [vgl. DOS 28 15 578]. Dihydropyridin-Derivate, die in 4-Position einen Phenyl-Ring tragen, der gegebenenfalls durch Halogen und/oder Cyano substituiert ist sind ebenfalls in allgemeinen Definitionen und teilweise auch als spezielle Einzelverbindungen beschrieben (vgl. DOS 19 63 188, DOS 21 17 573 und EP-A-0 26 317). Für keins der bisher bekannten Dihydropyridine ist jedoch in 4-Position der spezielle 2-Chlor-3-cyano-phenyl-Rest genannt. Dieser spezielle Substituent in 4-Position, der durch den ebenfalls neuen 2-Chlor-3-cyano-benzaldehyd über entsprechende Ringschlußreaktionen eingeführt wird, stellt ein wesentliches Element der vorliegenden Erfindung dar.

Die vorliegende Erfindung betrifft die Verbindung rac-Isopropyl-(2-methoxyethyl)-4-(2-chlor-3-cyano-phenyl)-1,4-dihydro-2,6-dimethylpyridin-3,5-dicarboxylat der Formel (I) 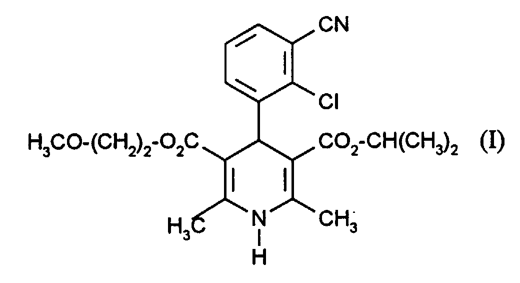 und deren Enantiomere.

Die Erfindung betrifft auch mehrere Verfahren zur Herstellung der erfindungsgemäßen Verbindung der Formel (I) , die dadurch gekennzeichnet sind, daß man
[A] 2-Chlor-3-cyano-benzaldehyd der Formel (II) 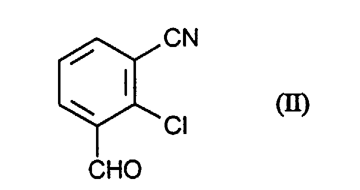 zunächst mit Acetessigsäure-2-methoxyethylester der Formel (III)

   H₃C-CO-CH₂-CO₂(CH₂)₂OCH₃ (III)

   gegebenenfalls unter Isolierung der entsprechenden Ylidenverbindung der Formel (IV) 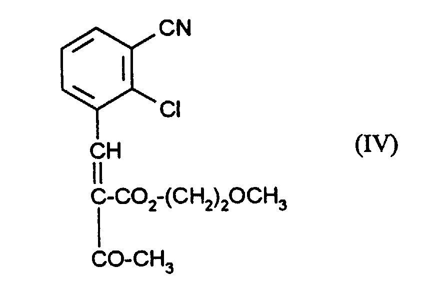 umsetzt und anschließend mit Acetessigsäureisopropylester der Formel (V)

   CH₃-CO-CH₂-CO₂-CH(CH₃)₂ (V)

   und mit Ammoniak oder Ammoniumsalzen,
   oder direkt mit dem aus Ammoniak und (V) herstellbaren Amino-2-butensäureisopropylester der Formel (VI) 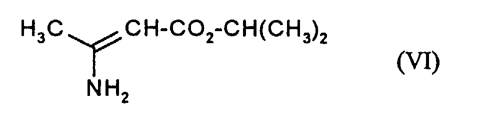 in inerten Lösemitteln umsetzt,
   oder
[B] 2-Chlor-3-cyano-benzaldehyd der Formel (II) zunächst mit der Verbindung der Formel (V), gegebenenfalls unter Isolierung der Ylidenverbindung der Formel (VII) 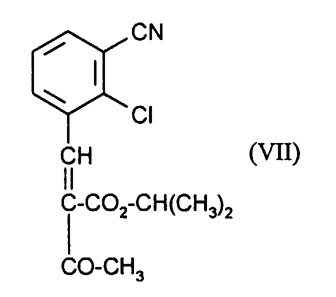 umsetzt und in einem nächsten Schritt mit der Verbindung der Formel (III) und mit Ammoniak oder Ammoniumsalzen oder direkt mit dem aus Ammoniak und (III) herstellbaren Amino-2-butensäure-2-methoxyethylester der Formel (VIII) 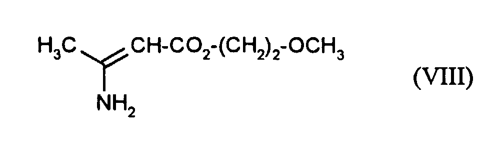 in inerten Lösemitteln umsetzt,
   oder
[C] Verbindungen der allgemeinen Formel (IX) 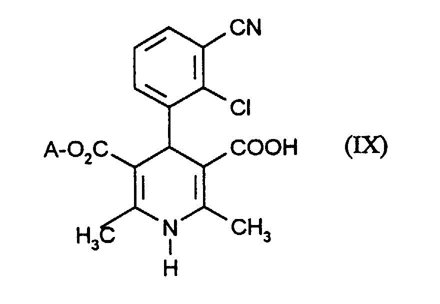 in welcher
   - A: für den Rest der Formel -CH(CH₃)₂ oder -(CH₂)₂-OCH₃ steht,
   in inerten Lösemitteln, gegebenenfalls in Anwesenheit einer Base, mit Hilfsstoffen zu aktivierten Carbonsäurederivaten, die gegebenenfalls isoliert werden, umsetzt und anschließend mit Alkoholen der Formel (X)

   E-OH (X)

   in welcher
   - E: in Abhängigkeit der Definition des Substituenten A entweder für die -CH(CH₃)₂- oder -(CH₂)₂-OCH₃-Gruppe steht,
   gegebenenfalls nach Überführung mit einer anorganischen Base in das Alkoholat,
   umsetzt,
   und zur Herstellung der Enantiomere, die entsprechenden optisch aktiven Carbonsäurederivate mit den entsprechenden Alkoholen umsetzt, oder
   das Racemat an chiralen stationären Phasen chromatographisch trennt.

Das erfindungsgemäße Verfahren kann durch folgendes Formelschema beispielhaft erläutert werden:

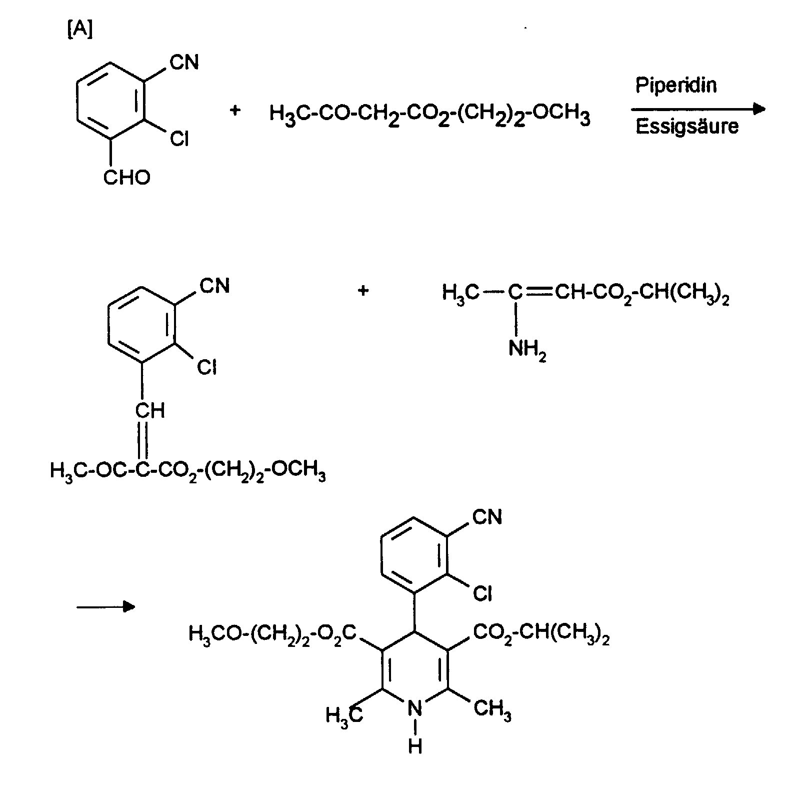

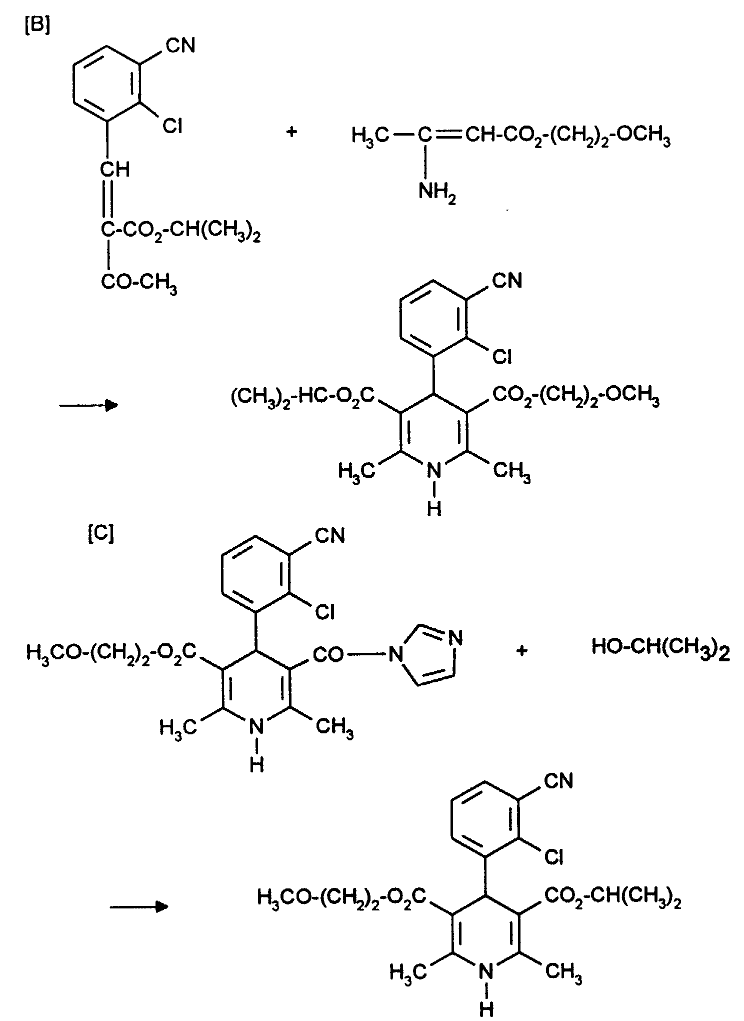

Als Lösemittel für die Verfahren [A] und [B] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt sind Isopropanol, Tetrahydrofuran, Methanol, Dioxan und Dimethylformamid.

Als Lösemittel für das Verfahren [C] eignen sich die oben aufgeführten Lösemittel mit Ausnahme der Alkohole und Essigsäure.

Als Basen für die Verfahren [A] und [B] eignen sich vorzugsweise cyclische Amine, wie beispielsweise Piperidin, C₁-C₃-Tri- und Dialkylamine, wie beispielsweise Di- und Triethylamin oder Pyridin oder Dimethylaminopyridin, insbesondere Piperidin, Dimethylaminopyridin und Pyridin.

Als Hilfsstoffe zur Aktivierung der Carbonsäure (IX) werden bevorzugt Kondensationsmittel eingesetzt. Bevorzugt werden hier die üblichen Kondensationsmittel wie Carbodiimide z.B. N,N'-Diethyl-, N,N'-Dipropyl-, N,N-Diisopropyl-, N,NN,N'-Dicyclohexylcarbodiimid, N-(3-Dimethylaminoisopropyl)-N-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat. Bevorzugt sind N,N-Dicyclohexylcarbodiimid und Carbonyldiimidazol.

Als Basen für die Variante [C] eignen sich Alkalicarbonate wie beispielsweise Natrium- oder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]undec-7-en (DBU) oder 1,5-Diazabicyclo[4.3.0]non-5-en (DBN). Bevorzugt ist Dimethylaminopyridin.

Die Base wird im allgemeinen in einer Menge von 0,01 mol bis 1 mol, bevorzugt von 0,05 mol bis 0,1 mol jeweils bezogen auf 1 mol der Verbindung der Formel (IX) eingesetzt.

Die Hilfsstoffe gemäß Variante [C] werden im allgemeinen in einer Menge von 1 mol bis 3 mol, bevorzugt von 1 mol bis 1,5 mol, jeweils bezogen auf 1 mol der Verbindung der Formel (IX) eingesetzt.

Als anorganische Basen gemäß [C] werden Alkalimetalle oder deren Hydride eingesetzt. Bevorzugt werden Natrium und Natriumhydrid. Die anorganischen Basen gemäß Verfahren [C] werden im allgemeinen in einer Menge von 0, 1 bis 10 mol, bevorzugt 0,2 bis 2 mol, bezogen auf 1 mol der Verbindung der Formel IV, eingesetzt.

Die Reaktionstemperatur für die Verfahren [A], [B] und [C] können in einem größeren Bereich variiert werden. Im allgemeinen arbeitet man in einem Bereich von -20°C bis 200°C, bevorzugt von 0°C bis 110°C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Enantiomerenreine Formen erhält man z.B. außerdem, indem man das Racemat auf Chromatographiesäulen mit chiraler stationärer Phase trennt.

Die reaktiven Säurederivate der allgemeinen Formel (IX) sind teilweise bekannt oder neu und können dann nach üblichen Methoden hergestellt werden.

Die Verbindungen der allgemeinen Formel (X) sind bekannt.

Die Ylidenverbindung der Formel (IV) ist neu und kann beispielsweise wie oben beschrieben hergestellt werden.

Die Ylidenverbindung der Formel (VII) ist neu und kann beispielsweise wie unter [A] beschrieben durch Umsetzung der Verbindung der Formel (V) mit 2-Chlor-3-cyano-benzaldehyd der Formel (II) hergestellt werden.

Die Verbindungen der Formeln (III), (V), (VI) und (VIII) sind an sich bekannt.

2-Chlor-3-Cyano-benzaldehyd der Formel (II) ist neu und kann beispielsweise hergestellt werden, indem man
2-Chlor-3-cyano-toluol der Formel (XI) 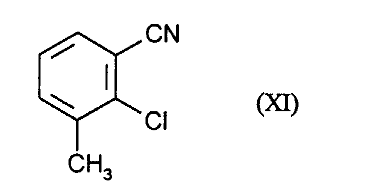 zunächst durch Bromierung in Anwesenheit eines Katalysators in inerten Lösemitteln und unter Schutzgasatmosphäre und Bestrahlung
in die Verbindung 2-Chlor-3-cyano-benzalbromid der Formel (XII) 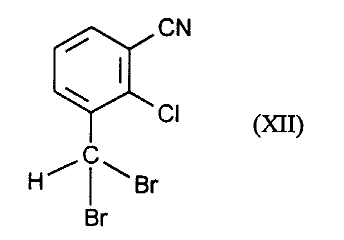 überführt,
und in einem zweiten Schritt das Rohprodukt in alkoholischer Lösung mit SilberSalzen versetzt und zum Aldehyd umsetzt.

Als Lösemittel für den ersten Schritt der Umsetzung eignen sich bevorzugt die oben aufgeführten Halogenkohlenwasserstoffe wie Dichlormethan oder Tetrachlorkohlenstoff. Besonders bevorzugt ist Tetrachlorkohlenstoff.

Als Bromierungsmittel eignen sich bevorzugt N-Bromsuccinimid oder Brom. Besonders bevorzugt ist N-Bromsuccinimid.

Als Lösemittel für den zweiten Schritt des Verfahrens eignen sich Wasser oder die oben aufgeführten Alkohole wie beispielsweise Methanol, Ethanol, Propanol oder Isopropanol oder deren Gemische. Bevorzugt ist Ethanol.

Als Katalysator eignen sich beispielswiese Azoisobutyronitril oder Bisbenzoylperoxid. Bevorzugt ist Azoisobutyronitril.

Der Katalysator wird im allgemeinen in einer Menge von 0,0005 mol bis 0,05 mol, bevorzugt von 0,005 mol bis 0,02 mol bezogen auf 1 mol der Verbindung der Formel (XI) eingesetzt.

N-Bromsuccinimid wird in einer Menge von 2 mol bis 5 mol, bevorzugt von 2,2 mol bis 3 mol bezogen auf 1 mol der Verbindung der Formel (XI) eingesetzt.

Die Bromierung wird in einem Temperaturbereich von 40°C bis zur jeweiligen Rückflußtemperatur des Lösemittels, bevorzugt bei der Rückflußtemperatur durchgeführt.

Die Überführung zum Aldehyd erfolgt in einem Temperaturbereich von 20°C bis 100°C, bevorzugt von 40°C bis 70°C.

Die Umsetzungen werden im allgemeinen bei Normaldruck durchgeführt. Es ist aber auch möglich bei Über- oder Unterdruck (z.B. in einem Bereich von 0,5 bis 5 bar) zu arbeiten.

Als Silber-Salze eignen sich im allgemeinen Salze wie Silbernitrat, Silbercarbonat oder Silbertetrafluoroborat. Bevorzugt ist Silbernitrat.

2-Chlor-3-cyano-toluol der Formel (XI) ist bekannt (CAS 15015-71-5).

2-Chlor-3-cyano-benzalbromid der Formel (XII) ist neu und kann beispielsweise wie oben beschrieben hergestellt werden.

Die vorstehenden Herstellungsverfahren sind lediglich zur Verdeutlichung angegeben. Die Herstellung der Verbindung der allgemeinen Formel (I) und deren Enantiomere ist nicht auf diese Verfahren beschränkt, sondern jede Modifikation dieser Verfahren und übliche alternative Verfahren sind in gleicher Weise für die Herstellung der erfindungsgemäßen Verbindung anwendbar.

Die erfindungsgemäße Verbindung und deren reine Enantiomere zeigen ein nicht vorhersehbares, wertvolles pharmakologisches Wirkspektrum. Sie beeinflußen positiv Lern- und Gedächtnisleistungen, wie ihre leistungsverbessernde Wirkung in typischen Lern- und Gedächtnismodellen wie Wasser-Labyrinth, Passives Vermeiden oder Reminiszenztests in automatisierten Skinner-Boxen demonstriert. Sie besitzen ein antidepressives Potential, wie ihre Wirksamkeit im Rattenschwimmtest nach Porsolt belegt.

### Bindungsassays:

Die Bindungsaffinitäten zu PN 200-110-Bindungsstellen im Rattenhirnen bzw. Rattenherzen wurden nach Rampe D.R., Mutledge A., Janis R.A., Triggle D.J.: Can. Journ. Physiol. Pharmacol. 65, (1987) 1452 bestimmt.

### Wasserlabyrinth:

Alte Wistar-Ratten werden an die Startposition in einem mit kaltem Wasser gefüllten, durch senkrechte Barrieren unterteilten Plastiktank gesetzt. Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichet. Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.

Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo ist Verkürzung der Zeit bis zum Erreichen des Ausstiegs, Verringerung der Zahl der Fehler und Vergrößerung der Zahl der Tiere,die den Ausstieg überhaupt finden.

### Rattenschwimmtest nach Porsolt

Während eines Vortests werden junge Ratten in einen Glaszylinder (40 cm hoch, 20 cm Durchmesser) gesetzt, der 17 cm hoch mit Wasser von 25 C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral despair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Die antidepressive Wirkung der Testsubstanzen zeigt sich in der Reduktion der Immobilitätsdauer im Vergleich zu den Placebowerten.

Die erfindungsgemäße Verbindung und ihre reinen Enantiomere sind bekannten Dihydropyridinen in ihrer Wirksamkeit des Lernens und des Gedächtnisses überlegen. So verbessert etwa (+)-Isopropyl-(2-methoxyethyl)-4-(2-chlor-3-cyanophenyl)-1,4-dihydro-2,6-dimethyl-pyridin-3,5-dicarboxylat im Tiermodell des Wasserlabyrinths (alte Ratten) im Bereich von 0,1 bis 1,0 mg /p.o. signifikant die Acquisition, während Nimodipin erst bei 15 mg/kg p.o. wirkt. Gleichzeitig zeigt die erfindungsgemäße Verbindung keine bzw. eine deutlich schwächere Blutdrucksenkung als bekannte Verbindungen wie z.B. Nimodipin. Im Tiermodell der normotensiven Ratte senkt Nimodipin den Blutdruck bei 30 mg/kg p.o. ungefähr 3x stärker als (+) Isopropyl-(2-methoxyethyl)-4-(2-chlor-3-cyano-phenyl)-1,4-dihydro-2,6-dimethyl-pyridin-3,5-dicarboxylat; gleiches wird im Tiermodell der spontan hypertensiven Ratte (3 und 10 mg/kg p.o.) gefunden. Es ist daher bei der erfindungsgemäßen Verbindung mit einer potenteren spezifischen ZNS-Wirkung bei geringerer - unerwünschter - Wirkung auf das Herz-Kreislaufsystem zu rechnen.

Aufgrund ihrer pharmakologischen Eigenschaften können die erfindungsgemäße Verbindung und ihre reinen Enantiomere für die Herstellung von Arzneimitteln zur Behandlung von zentral degenerativen Erkrankungen eingesetzt werden, wie sie z.B. auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignet sie sich zur Behandlung von Hirnleistungsstörungen im Alter, des hirnorganischen Psychosyndroms (HOPS, Organic brain syndrom, OBS) und von altersbedingten Gedächtnisstörungen (age associated memory impairment, AAMI).

Sie ist wertvoll zur Prophylaxe und zur Bekämpfung der Folgen cerebraler Durchblutungsstörungen wie cerebraler Ischämien, Schlaganfällen und von Subarachnoidalblutungen.

Sie ist geeignet zur Behandlung von Depressionen und der Manie. Weitere Anwendungsgebiete sind die Behandlung von Migräne, von Neuropathien, die z.B. durch metabolische Erkrankungen wie Diabetes mellitus, Traumata, Vergiftungen, Mikroorganismen oder Autoimmunerkrankungen verursacht werden, von Suchterkrankungen und Entzugserscheinungen.

Zur vorliegenden Erfindung gehören auch pharmazeutische Zubereitungen, die neben inerten, nicht-toxischen, pharmazeutisch geeigneten Hilfs- und Trägerstoffen die Verbindung der Formel (I) enthalten, sowie Verfahren zur Herstellung dieser Zubereitungen.

Der Wirkstoff der Formel (I) soll in diesen Zubereitungen in einer Konzentration von 0,1 bis 99,5 Gew.-%, bevorzugt von 0,5 bis 95 Gew.-% der Gesamtmischung vorhanden sein.

Neben dem Wirkstoff der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen ist es vorteilhaft, den Wirkstoff der Formel (I) in Gesamtmengen von etwa 0,01 bis etwa 50 mg/kg, bevorzugt in Gesamtmengen von etwa 0,1 mg/kg bis 10 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung oder mit Molybdatophosphorsäurelösung.

Die Flash-Chromatographie wurde auf Kieselgel 60, 0,040 -0,064 mm, Fa. E. Merck, durchgeführt. Elution mit Lösemittelgradienten bedeutet: Beginnend mit der reinen, unpolaren Lösemittelgemischkomponente wird in einem steigenden Ausmaß die polare Laufmittelkomponente zugemischt, bis das gewünschte Produkt eluiert wird (DC-Kontrolle). Bei allen Produkten wurde bei zuletzt ca. 0,1 Torr das Lösemittel abdestilliert.

### Ausgangsverbindungen

### Beispiel I

### 2-Acetyl-3(2-chlor-3-cyano)-2-propensäure-2-methoxyethylester

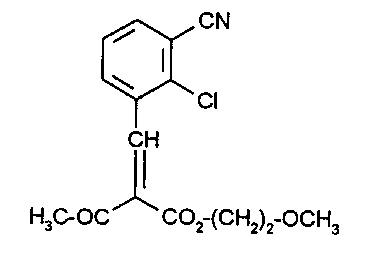

Zu 13,0 g (78,5 mmol) 2-Chlor-3-cyanobenzaldehyd und 13,8 g(86 mmol) Acetessigsäure-2-methoxyethylester in 130 ml Isopropanol werden 0,5 ml Piperidin und 0,3 ml Eisessig, gelöst in 4 ml Isopropanol, gegeben. Nach Rühren über Nacht bei 40°C wird eingeengt, in Toluol aufgenommen und über Kieselgel K₆₀ filtriert (Lösemittel: Toluol / Essigestergradient 1:0 bis 10:1). Nach Eindampfen erhält man 16,3 g gelbliches Öl, das ohne weitere Reinigung weiter umgesetzt wird.

### Beispiel II

### 2-Chlor-3-cyano-benzalbromid

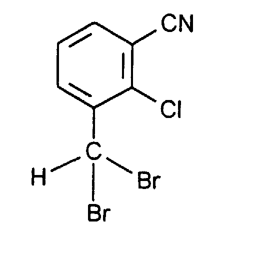

100 g (0,66 mol) 2-Chlor-3-cyano-toluol (CAS 15013-71-5) werden unter Argon in 1,0 1 Tetrachlorkohlenstoff gelöst und mit 280 g (1,6 mol) N-Bromsuccinimid und 0,65 g Azoisobutyronitril versetzt. Die Mischung wird unter heftigem Rühren zum Rückfluß erhitzt; dabei wird mit einer Lampe (300 W, Osram Ultra-Vitalux^{R}) bestrahlt. Nach 21 h und nach 36 h werden nochmals je 50 g N-Bromsuccinimid und 0,5 g Azoisobutyronitril zugegeben. Die Reaktion wird nach insgesamt 60 h beendet. Nach Abkühlen wird von Ungelöstem abfiltriert; der Niederschlag wird mehrmals mit Tetrachlorkohlenstoff nachgewaschen und verworfen. Nach Einengen des Filtrats erhält man so 180 g der Titelverbindung als Rohprodukt (enthält nach HPLC ca. 2% entsprechendes Benzylbromid und 1,2% entsprechendes Benzotribromid).

### Beispiel III

### 2-Chlor-3-cyano-benzaldehyd

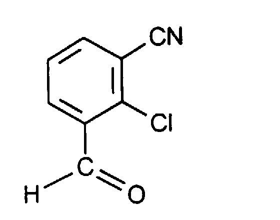

Das Rohprodukt der Verbindung aus Beispiel II wird in 1,5 1 95% Ethanol gelöst und auf 60°C erwärmt. Hierzu wird bei dieser Temperatur eine Lösung von 186 g (1,1 mol) Silbernitrat in 380 ml Wasser, die mit 750 ml Ethanol versetzt wurde, rasch zugetropft. Nach beendeter Zugabe wird eine Stunde nachgerührt und anschließend mit 50 ml gesättigter Kochsalzlösung versetzt. Nach Abkühlen wird von den Silbersalzen abfiltriert, und das Filtrat eingeengt. Der Rückstand wird in Methylenchlorid aufgenommen und durch Filtration über eine kurze Kieselgelsäule gereinigt (Laufmittel: Dichlormethan). Nach Einengen der Produktfraktionen erhält man 88 g (81%) der Titelverbindung.
Schmp.: 86-89°C
¹H-NMR (CHCl₃): δ = 7,55 ppm (t, 1H); 7,95 (dd, 1H); 8,20 (dd, 1H); 10,5 (s, 1H).

### Beispiel 1

### rac-Isopropyl-(2-methoxyethyl)-4-(2-chlor-3-cyano-phenyl)-1 ,4-dihydro-2,6-dimethyl-pyridin-3,5-dicarboxylat

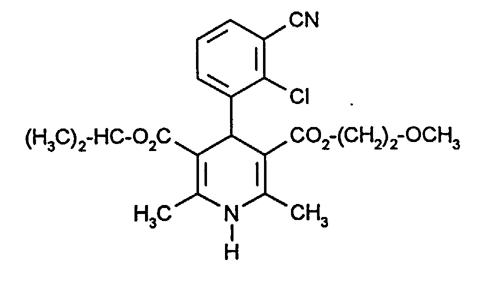

18,6 g (60,5 mmol) der Verbindung aus Beispiel I und 8,7 g (60,5 mmol) Amino-2-butensäureisopropylester in 250 ml Isopropanol werden 4 h auf Rückfluß erhitzt. Nach Aufkonzentrieren erhält man einen Feststoff, der mit Ether digeriert wird (18,1 g). Dieser wird in 180 ml Toluol in der Siedehitze mit der gleichen Menge Cyclohexan versetzt. Nach Abkühlen auf Raumtemperatur erhält man Kristalle, die bei 80°C im Umlufttrockenschrank getrocknet werden.
Ausbeute: 17,6 g (67%)
Schmp.: 148-149°C

### Beispiel 2 und Beispiel 3

### (+)-Isopropyl-(2-methoxyethyl)-4-(2-chlor-3-cyanophenyl)-1,4-dihydro-2,6-dimethylpyridin-3,5-dicarboxylat (-)-Isopropyl-(2-methoxyethyl)4-(2-chlor-3-cyano-phenyl)-1,4-dihydro-2,6-dimethylpyridin-3,5-dicarboxylat

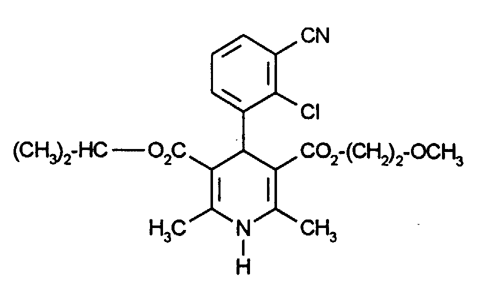

Die reinen Enantiomeren des Beispiels 1 können durch Chromatographie auf chiralen Säulen (z.B. Chiracel ^{R} OD-H, Laufmittel 95% n-Heptan / 5% Gemisch von 1% Wasser / 0,2% Trifluoressigsäure in Ethanol) erhalten werden.

### Beispiel 2:

Fp.: 138-140°C
α ²⁰_{D} = +13,9 (c=1, CHCl₃)

### Beispiel 3:

Fp.: 138-140°C
α ²⁰_{D} = -12,1 (c=0,9, CHCl₃)

## Patentansprüche

1. Isopropyl-(2-methoxyethyl)-4-(2-chlor-3-cyano-phenyl)-1,4-dihydro-2,6-dimethyl-pyridin-3,5-dicarboxylat als Racemat und seine enantiomeren Formen.

2. Verfahren zur Herstellung der Verbindung gemäß Anspruch 1, dadurch gekennzeichnet, daß man
[A] 2-Chlor-3-cyano-benzaldehyd der Formel (II) 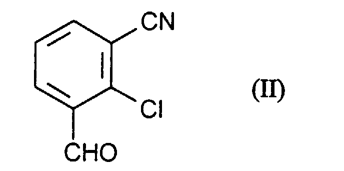 zunächst mit Acetessigsäure-2-methoxyethylester der Formel (III)
H₃C-CO-CH₂-CO₂(CH₂)₂OCH₃ (III)
gegebenenfalls unter Isolierung der entsprechenden Ylidenverbindung der Formel (IV) 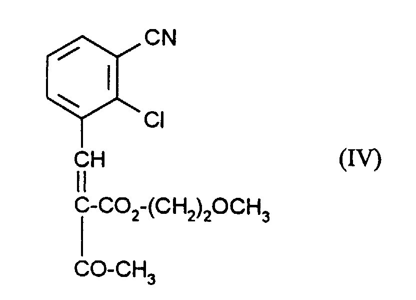 umsetzt und anschließend mit Acetessigsäureisopropylester der Formel (V)
CH₃-CO-CH₂-CO₂-CH(CH₃)₂ (V)
und mit Ammoniak oder Ammoniumsalzen,
oder direkt mit dem aus Ammoniak und (V) herstellbaren Amino-2-butensäureisopropylester der Formel (VI) 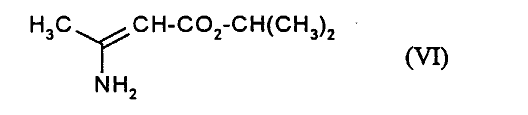 in inerten Lösemitteln umsetzt,
oder
[B] 2-Chlor-3-cyano-benzaldehyd der Formel (II) zunächst mit der Verbindung der Formel (V), gegebenenfalls unter Isolierung der Ylidenverbindung der Formel (VII) 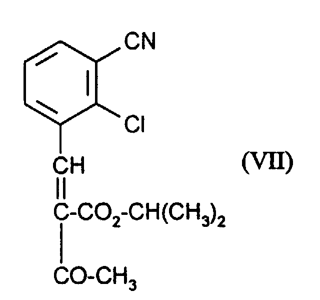 umsetzt und in einem nächsten Schritt mit der Verbindung der Formel (III) und mit Ammoniak oder Ammoniumsalzen oder direkt mit dem aus Ammoniak und (III) herstellbaren Amino-2-butensäue-2-methoxyethylester der Formel (VIII) 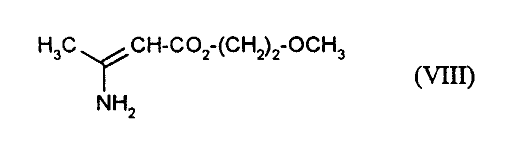 in inerten Lösemitteln umsetzt,
oder
[C] Verbindungen der allgemeinen Formel (IX) 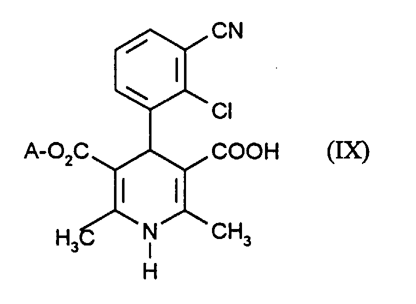 in welcher
A für den Rest der Formel -CH(CH₃)₂ oder -(CH₂)₂-OCH₃ steht,
in inerten Lösemitteln, gegebenenfalls in Anwesenheit einer Base, mit Hilfsstoffen zu aktivierten Carbonsäurederivaten, die gegebenenfalls isoliert werden, umsetzt und anschließend mit Alkoholen der Formel (X)
E-OH (X)
in welcher
E in Abhängigkeit der Definition des Substituenten A entweder für die
-CH(CH₃)₂- oder -(CH₂)₂-OCH₃-Gruppe
steht,
gegebenenfalls nach Überführung in das Alkoholat mit einer anorganischen Base,
umsetzt,
und zur Herstellung der Enantiomere, die entsprechenden optisch aktiven Carbonsäurederivate mit den entsprechenden Alkoholen umsetzt, oder
das Racemat an chiralen stationären Phasen chromatographisch trennt.

3. Arzneimittel enthaltend eine Verbindung gemäß Anspruch 1.

4. Verfahren zur Herstellung von Arzneimitteln, dadurch gekennzeichnet, daß man eine Verbindung gemäß Anspruch 1, gegebenenfalls mit üblichen Hilfs- und Trägerstoffen in eine geeignete Applikationsform überführt.

5. Verwendung der Verbindungen gemäß Anspruch 1 zur Herstellung eines Arzneimittels zur Bekämpfung von Erkrankungen.

6. Verwendung der Verbindungen gemäß Anspruch 1, bei der Herstellung von Arzneimitteln zur Bekämpfung von neuronalen und cerebralen Erkrankungen.

7. 2-Chlor-3-cyano-benzaldehyd.

8. Verfahren zur Herstellung von 2-Chlor-3-cyano-benzaldehyd, dadurch gekennzeichnet, daß man 2-Chlor-3-cyano-toluol in Anwesenheit eines Bromierungsmittels und eines Katalysators in inerten Lösemitteln zum entsprechenden Benzalbromid umsetzt, und dieses in Lösemitteln in Gegenwart von Silbersalz zum Aldehyd umsetzt.

## Claims

1. Isopropyl 2-methoxyethyl 4-(2-chloro-3-cyanophenyl)-1,4-dihydro-2,6-dimethyl-pyridine-3,5-dicarboxylate as a racemate and its enantiomeric forms.

2. Process for the preparation of the compound according to Claim 1, characterized in that
[A] 2-chloro-3-cyano-benzaldehyde of the formula (II) 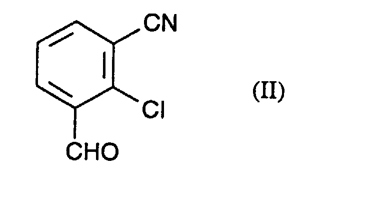 is reacted first with 2-methoxyethyl acetoacetate of the formula (III)
H₃C-CO-CH₂-CO₂(CH₂)₂OCH₃ (III)
if appropriate with isolation of the corresponding ylidene compound of the formula (IV) 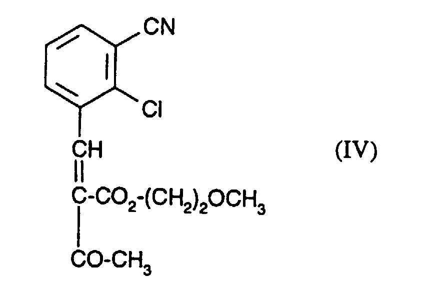 and subsequently with isopropyl acetoacetate of the formula (V)
CH₃-CO-CH₂-CO₂-CH(CH₃)₂ (V)
and with ammonia or ammonium salts,
or directly with the isopropyl amino-2-butenoate which can be prepared from ammonia and (V), of the formula (VI) 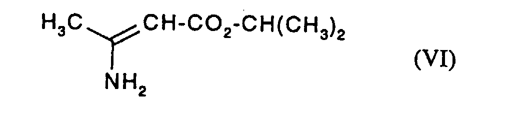 in inert solvents,
or
[B] 2-chloro-3-cyano-benzaldehyde of the formula (II) is reacted first with the compound of the formula (V), if appropriate with isolation of the ylidene compound of the formula (VII) 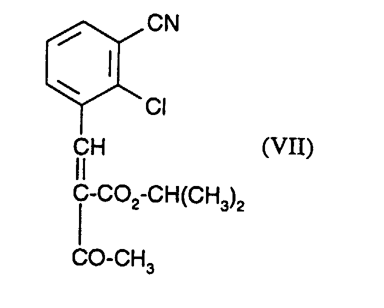 and in a next step with the compound of the formula (III) and with ammonia or ammonium salts or directly with the 2-methoxyethyl amino-2-butenoate which can be prepared from ammonia and (III), of the formula (VIII) 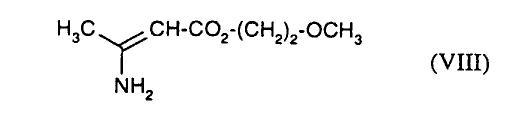 in inert solvents,
or
[C] compounds of the general formula (IX) 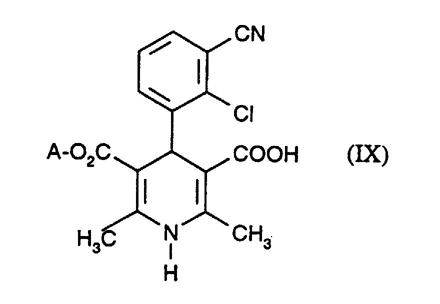 in which
A represents the radical of the formula -CH(CH₃)₂ or - (CH₂)₂-OCH₃,
are reacted in inert solvents, if appropriate in the presence of a base, with auxiliaries to give activated carboxylic acid derivatives, which are optionally isolated, and then reacted with alcohols of the formula (X)
E-OH (X)
in which
E depending on the definition of the substituent A, either represents the -CH(CH₃)₂- or -(CH₂)₂-OCH₃-group,
if appropriate after conversion to the alkoxide using an inorganic base,
and for the preparation of the enantiomers, the appropriate optically active carboxylic acid derivatives are reacted with the appropriate alcohols, or
the racemate is separated by chromatography on chiral stationary phases.

3. Medicament containing a compound according to Claim 1.

4. Process for the production of medicaments, characterized in that a compound according to Claim 1 is converted into a suitable administration form, if appropriate using customary auxiliaries and excipients.

5. Use of the compounds according to Claim 1 for the production of a medicament for the control of disorders.

6. Use of the compound according to Claim 1 in the production of medicaments for the control of neuronal and cerebral disorders.

7. 2-chloro-3-cyano-benzaldehyde.

8. Process for the preparation of 2-chloro-3-cyanobenzaldehyde, characterized in that 2-chloro-3-cyano-toluene is reacted in the presence of a brominating agent and of a catalyst in inert solvents to give the corresponding benzal bromide, and this is reacted in solvents in the presence of silver salt to give the aldehyde.

## Revendications

1. 4-(2-chloro-3-cyano-phényl)-1,4-dihydro-2,6-diméthyl-pyridine-3,5-dicarboxylate d'isopropyle et 2-méthoxyéthyle sous forme racémique, ainsi que ses formes énantiomères.

2. Procédé pour la préparation du composé selon la revendication 1, caractérisé en ce que
[A] on fait réagir du 2-chloro-3-cyanobenzaldéhyde répondant à la formule (II) 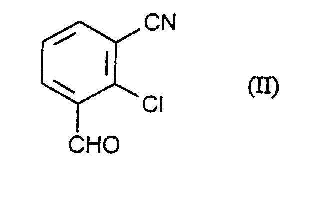 d'abord avec de l'ester 2-méthoxyéthylique de l'acide acétoacétique répondant à la formule (III)
H₃C-CO-CH₂-CO₂(CH₂)₂OCH₃ (III)
le cas échéant en isolant le composé d'ylidène correspondant de formule (IV) 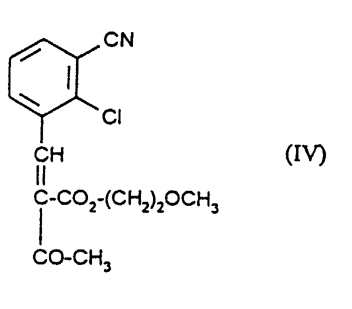 et ensuite, avec l'ester isopropylique de l'acide acétoacétique répondant à la formule (V)
CH₃-CO-CH₂-CO₂-CH(CH₃)₂ (V)
et avec de l'ammoniac ou avec des sels d'ammonium,
ou bien on le fait réagir directement avec l'ester isopropylique de l'acide amino-2-buténoïque que l'on obtient à partir d'ammoniac et de (V), et qui répond à la formule (VI) 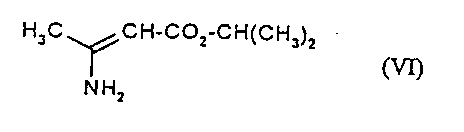 dans des solvants inertes,
ou
[B] on fait réagir du 2-chloro-3-cyanobenzaldéhyde répondant à la formule (II) d'abord avec le composé de formule (V), le cas échéant en isolant le composé d'ylidène de formule (VII) 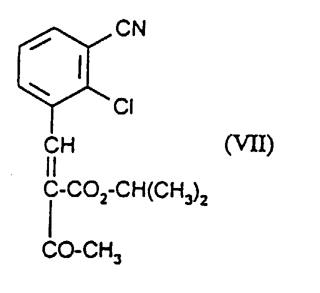 et dans une étape ultérieure, on le fait réagir avec le composé de formule (III) et avec de l'ammoniac ou avec des sels d'ammonium, ou bien on le fait réagir directement avec l'ester 2-méthoxyéthylique de l'acide amino-2-buténoïque que l'on obtient à partir d'ammoniac et de (III), et qui répond à la formule (VIII) 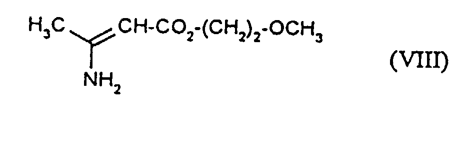 dans des solvants inertes,
ou
[C] on fait réagir des composés répondant à la formule générale (IX) 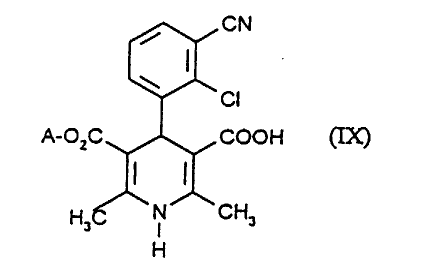 dans laquelle
A représente le radical de formule -CH(CH₃)₂ ou -(CH₂)₂-OCH₃,
dans des solvants inertes, le cas échéant en présence d'une base, avec des adjuvants pour obtenir des dérivés activés d'acides carboxyliques que l'on isole le cas échéant, et ensuite avec des alcools de formule (X)
E-OH (X)
dans laquelle
E représente, en fonction de la définition du substituant A, soit le groupe -CH(CH₃)₂, soit le groupe -(CH₂)₂-OCH₃,
le cas échéant après transformation en alcoolate avec une base inorganique,
et pour la préparation des énantiomères, on fait réagir les dérivés d'acides carboxyliques optiquement actifs correspondants avec les alcools correspondants, ou bien
on dédouble le racémate par voie chromatographique sur des phases stationnaires chirales.

3. Médicament contenant un composé selon la revendication 1.

4. Procédé pour la préparation de médicaments, caractérisé en ce qu'on transforme un composé selon la revendication 1, le cas échéant avec des substances auxiliaires de support habituelles, en une forme d'application appropriée.

5. Utilisation des composés selon la revendication 1 pour la préparation d'un médicament destiné à lutter contre des maladies.

6. Utilisation des composés selon la revendication 1, dans la préparation de médicaments destinés à lutter contre des maladies nerveuses et cérébrales.

7. 2-chloro-3-cyano-benzaldéhyde.

8. Procédé pour la préparation du 2-chloro-3-cyano-benzaldéhyde, caractérisé en ce qu'on fait réagir du 2-chloro-3-cyano-toluène en présence d'un agent de bromation et d'un catalyseur dans des solvants inertes pour obtenir le benzalbromure correspondant que l'on fait réagir dans des solvants en présence d'un sel d'argent pour obtenir l'aldéhyde.
